# EUROPÄISCHE PATENTANMELDUNG

(11) **EP 1 671 672 A1**
(43) Veröffentlichungstag der Anmeldung: **21.06.2006**
(21) Anmeldenummer: 05026907.5
(22) Anmeldetag: 08.12.2005
(51) Int. Cl.: A61N 2/02

(54) **Magnetischer Neurostimulator**

(30) Priorität: 20.12.2004 DE 102004061293
(71) Anmelder: MAG & MORE GmbH, 80333 München (DE)
(72) Erfinder: Wendicke, Kerstin, 80339 München (DE); Weyh, Thomas, Dr., 80803 München (DE); Zantow, Hannes, 80339 München (DE)
(74) Vertreter: Kirschner, Klaus Dieter

(57) **Zusammenfassung**

Ein erfindungsgemäßer magnetischer Neurostimulator mit einer Ausgangswert-Überwachungsschaltung beinhaltet einen Schwingkreis mit einer Stimulationsspule (L_{S}) zum Erzeugen eines Magnetfelds und mit einer Pulsquelle (C_{P}), die eine Spannung an die Stimulationsspule (L_{S}) abgibt; einen Pulsgenerator (PG) zum Generieren eines Pulsauslösersignals; einen Pulsauslöserschalter (PA), der in Abhängigkeit des Pulsauslösersignals den Schwingkreis schliesst oder unterbricht; einen Spannungsbeschränkungs-Wahlschalter (VWS) zum Einstellen eines Spannungs-Grenzwerts und/oder einen Pulsfrequenzbeschränkungs-Wahlschalter (fWS) zum Einstellen eines Pulsfrequenz-Grenzwerts; eine Ausgangswert-Überwachungsschaltung (2), die einen optischen oder akustischen Alarm ausgibt und/oder die den Schwingkreis unterbricht, wenn eine an der Pulsquelle (C_{P}) gemessene Spannung den Spannungs-Grenzwert übersteigt und/oder wenn eine von dem Pulsgenerator (PG) vorgegebene Pulsfrequenz den Pulsfrequenz-Grenzwert übersteigt.

## Beschreibung

Die magnetische Neurostimulation ist eine nicht-invasive, nahezu schmerzfreie Methode, mit der Nerven eines Patienten durch ein zeitlich verändertes Magnetfeld nach dem physikalischen Prinzip der Induktion in ihrer elektrischen Aktivität beeinflusst werden können. Dabei kommt ein magnetischer Neurostimulator zum Einsatz, der eine beispielsweise mit einem Griff ausgestattete Stimulationsspule aufweist, die mit Strompulsen gespeist wird. Diese Stimulationsspule wird vom behandelnden Arzt über dem Zielgebiet, beispielsweise dem Kortex oder der Peripherie des Patienten platziert. Dabei werden die Stimulationsparameter Spannungsintensität und Pulsfrequenz je nach Behandlungsziel vom behandelnden Arzt geeignet gewählt. Diese Parameterauswahl erfolgt über entsprechende Regler oder Schalter auf einer Frontseite eines Leistungsteil-Gehäuses, das mit der Stimulationsspule verbunden ist und die Steuerelektronik beinhaltet. Durch eine geeignete Auswahl der Stimulationsparameter kann eine gezielte Stimulation von Kortexarealen mit einer transkraniellen Magnetstimulation TMS erreicht werden.

Ein solcher magnetischer Neurostimulator birgt das Risiko, dass er einen ungewollten epileptischen Anfall des Patienten auslöst.

Es ist daher Aufgabe der vorliegenden Erfindung, einen magnetischen Neurostimulator sowie ein Verfahren zur magnetischen Neurostimulation bereitzustellen, bei denen das Risiko eines ungewollten epileptischen Anfalls des Patienten vermindert werden kann.

Diese Aufgabe wird durch den Gegenstand der unabhängigen Patentansprüche gelöst. Vorteilhafte Weiterbildungen ergeben sich aus den abhängigen Ansprüchen.

Gemäß der Erfindung wird ein magnetischer Neurostimulator mit einer Ausgangswert-Überwachungsschaltung bereitgestellt, der in der Lage ist, die beiden wichtigen Ausgabeparameter Spannungsintensität und Pulsfrequenz bzw. Wiederholfrequenz der Spannungspulse einer kontinuierlichen Prüfung zu unterziehen. Dafür weist der magnetische Neurostimulator einen Schwingkreis mit einer Stimulationsspule zum Erzeugen eines Magnetfelds zum Stimulieren eines Kortex oder einer Peripherie und mit einer Pulsquelle auf, die eine Spannung an die Stimulationsspule abgibt. Des Weiteren weist ein solcher magnetischer Neurostimulator einen Pulsgenerator zum Generieren eines Pulsauslösersignals sowie einen Pulsauslöserschalter auf, der in Abhängigkeit des Pulsauslösersignals den Schwingkreis schließt oder unterbricht. Erfindungsgemäß weist ein derartiger magnetischer Neurostimulator einen Spannungsbeschränkungs-Wahlschalter auf, mit dem ein Benutzer einen Spannungs-Grenzwert individuell einstellen kann. Alternativ oder zusätzlich dazu kann ein Pulsfrequenzbeschränkungs-Wahlschalter vorgesehen sein, mit dem der Benutzer einen Pulsfrequenz-Grenzwert individuell einstellen kann.

Ferner verfügt der magnetische Neurostimulator über eine Ausgangswert-Überwachungsschaltung, die einen optischen oder akustischen Alarm ausgibt und/oder die den Schwingkreis unterbricht, wenn eine an der Pulsquelle gemessene Spannung den Spannungs-Grenzwert übersteigt. Alternativ oder zusätzlich dazu gibt die Ausgangswert-Überwachungsschaltung einen optischen und/oder akustischen Alarm aus oder unterbricht den Schwingkreis dann, wenn eine von dem Pulsgenerator vorgegebene Pulsfrequenz dem Pulsfrequenz-Grenzwert übersteigt. Die Ausgangswert-Überwachungsschaltung kann also in einfachen Ausführungsformen so ausgestaltet sein, dass sie im Falle einer festgestellten zu hohen Spannung oder Pulsfrequenz entweder eine Rückmeldung an den Benutzer in Form eines akustischen und/oder optischen Alarm ausgibt oder den Schwingkreis ohne eine Rückmeldung unterbricht. Eine solche Ausgangswert-Überwachungsschaltung benötigt weniger Schaltungselemente. Es ist jedoch komfortabler, wenn eine solche Ausgangswert-Überwachungsschaltung im Falle einer festgestellten zu hohen Spannung oder Pulsfrequenz sowohl eine Rückmeldung in Form eines Alarms als auch eine Schwingkreisunterbrechung vornimmt.

Gemäß einem Grundgedanken der Erfindung können durch einen magnetischen Neurostimulator mit einer derartigen Ausgangswert-Überwachungsschaltung eine ungewollt hohe Reizintensität oder Pulsfrequenz zuverlässig erkannt und vermieden werden. Dadurch kann das Risiko bei der magnetischen Neurostimulation, insbesondere die Wahrscheinlichkeit von ungewollt ausgelösten epileptischen Anfällen beträchtlich verringert werden. Die erfindungsgemäße Ausgangswert-Überwachungsschaltung signalisiert zu hohe Spannungs- oder Pulsfrequenzwerte und/oder unterbricht die Abgabe von Stimulationspulsen unabhängig davon, ob die Parameter auf der Gerätefrontseite falsch eingestellt oder über eine externe Geräteschnittstelle falsch vorgegeben werden, sowie unabhängig davon, ob ein interner Gerätefehler, insbesondere ein Hard-/Softwarefehler aufgetreten ist.

Durch die separate Einstellbarkeit des Spannungs- und/oder Pulsfrequenz-Grenzwertes kann der magnetische Neurostimulator auf einfache Weise und individuell an die Bedürfnisse verschiedener Patienten angepasst werden.

In einfacheren Ausführungsformen kann der magnetische Neurostimulator jeweils nur mit einem Spannungsbeschränkungs-Wahlschalter oder mit einem Pulsfrequenzbeschränkungs-Wahlschalter ausgestattet werden. Dabei erfolgt entweder eine Überwachung der an der Pulsquelle gemessenen Spannung, oder eine Überwachung der von dem Pulsgenerator vorgegebenen Pulsfrequenz. Eine komfortablere Ausgangswertüberwachung ergibt sich jedoch, wenn der magnetische Neurostimulator sowohl mit einem Spannungsbeschränkungs- als auch mit einem Pulsfrequenzbeschränkungs-Wahlschalter ausgestattet ist. In diesem Fall erfolgt sowohl eine Überwachung der an der Pulsquelle gemessenen Spannung als auch eine Überwachung der von dem Pulsgenerator vorgegebenen Pulsfrequenz

Anstelle der im nachfolgenden Ausführungsbeispiel realisierten sechs verschiedenen Schaltstellungen ist auch eine stufenlose Vorgabe der Grenzwerte denkbar.

Gemäß einer ersten Ausführungsform der Erfindung umfasst die Ausgangswertüberwachungs-Schaltung jeweils eine Spannungsüberwachungs-Teilschaltung zum Erfassen und zum Aufbereiten der Spannung der Pulsquelle und eine Pulsfrequenzüberwachungs-Teilschaltung. Erfindungsgemäß werden dabei komplett unabhängige Kontroller für die beiden wichtigen Output-Parameter Stimulationsintensität und Wiederholfrequenz der Pulse vorgesehen.

Eine kontinuierliche Überprüfung der Spannung kann dabei durch einen Spannungskomparator erfolgen, dessen erster Eingang einen Spannungswert von der Pulsquelle erhält und dessen zweiter Eingang mit dem Spannungsbeschränkungs-Wahlschalter in Verbindung steht.

Eine besonders einfach zu realisierende Hardwareschaltung der Spannungsüberwachungs-Teilschaltung ist dann gegeben, wenn sie zum Aufbereiten der gemessenen Spannungswerte wenigstens einen an der Pulsquelle ansetzenden Hochspannungsteiler, wenigstens einen danach angeordneten Differenzverstärker und wenigstens ein danach angeordnetes Betragsbildungsschaltelement aufweist, das mit dem ersten Eingang des Spannungskomparators verbunden ist. Ein weiteres optional vorsehbares Schaltelement ist ein Tiefpassfilter, der sich an das Betragsbildungsschaltelement anschließt . Wenn ein Tiefpassfilter vorgesehen wird, ist dieser anstelle des Betragsbildungsschaltelements mit dem ersten Eingang des Spannungskomparators verbunden.

Um die technische Zuverlässigkeit der Spannungsüberwachungs-Teilschaltung zu erhöhen, können jeweils zwei redundante, parallel angeordnete Schaltungsarme vorgesehen werden, die jeweils einen Hochspannungsteiler, einen Differenzverstärker, ein Betragsbildungsschaltelement, einen Tiefpassfilter und einen Spannungskomparator aufweisen. Dabei kontrollieren zwei identische, komplett redundant aufgebaute Hardwareschaltungen die Anforderungen der Steuerelektronik des Gerätes an dem Leistungsteil.

Um die technische Zuverlässigkeit weiter zu erhöhen, kann nach den Betragsbildungs-Schaltelementen eine Komparator-Schaltung zur Plausibilitätsprüfung der Spannungswerte angeordnet werden, welche die von den parallel angeordneten Hochspannungsteilern, Differenzverstärkern und Betragsbildungsschaltelementen gemessenen und aufbereiteten Spannungswerte vergleicht, optional glättet und den Schwingkreis unterbricht, falls eine Abweichung oder ein Fehler festgestellt wird.

Eine kontinuierliche Überprüfung der Pulsfrequenz kann beispielsweise dann erfolgen, wenn die Pulsfrequenzüberwachungs-Teilschaltung wenigstens einen Pulsfrequenzkomparator aufweist, dessen erster Eingang von dem Pulsgenerator einen Stimulationspuls erhält und dessen zweiter Eingang mit dem Pulsfrequenzbeschränkungs-Wahlschalter in Verbindung steht.

Zur Erhöhung der technischen Zuverlässigkeit können auch die Pulsfrequenz-Komparatoren redundant ausgelegt werden.

Gemäß einer vorteilhaften Weiterbildung der Erfindung sind die Spannungsüberwachungs- und die Pulsfrequenzüberwachungs-Teilschaltungen über wenigstens ein Oder-Schaltelement mit wenigstens einem akustischen oder optischen Alarmgeber verbunden, der durch einen einen Fehler signalisierenden Ausgangswert des Oder-Schaltelements aktiviert wird. Wenn die Spannungsüberwachungs- und die Pulsfrequenzüberwachungs-Teilschaltung redundant ausgelegt sind, sind vorzugsweise zwei Oder-Schaltelemente vorzusehen.

Nach dem Oder-Schaltelement bzw. nach den Oder-Schaltelementen kann jeweils ein Halteglied vorgesehen sein, welches das einen Fehler signalisierende Ausgangssignal des betreffenden Oder-Schaltelementes für einen vorbestimmten Zeitraum hält. Dadurch werden die Alarmgeber für einen vorbestimmten, nicht zu klein bemessenen Zeitraums aktiviert, sodass sie von dem Benutzer auf jeden Fall wahrgenommen werden. Durch die Halteglieder wird sichergestellt, dass der akustische und optische Alarm sowie die Blockierung der Pulsabgabe für mindestens fünf Sekunden anhält. Bei Fortdauern des Fehlerzustandes wird dieser Zustand entsprechend länger beibehalten.

Durch Anordnen eines Verzögerungselementes nach dem Pulsgenerator, das den Signalfluss um ein bestimmtes Zeitintervall verzögert, wird sichergestellt, dass die Pulsfrequenzüberwachungs-Teilschaltung ausreichend Zeit hat, den vom Pulsgenerator erzeugten Puls zu überprüfen und im Falle der Unzulässigkeit bzw. des Überschreitens der zulässigen Pulsfrequenz zu blockieren. Dafür kann nach dem Verzögerungselement wenigstens ein Schließschalter angeordnet werden, der den Schwingkreis unterbricht, wenn er ein Fehlersignal des bzw. eines Oder-Schaltelementes erhält.

Um die Bedienungssicherheit des erfindungsgemäßen magnetischen Neurostimulators zu erhöhen, können noch ein Fußtaster oder ein Spulentaster integriert und deren Betätigungssignale in die Pulsfrequenzüberwachungs-Teilschaltung eingeschleift werden. In diesem Fall unterbricht der bzw. ein Schließschalter den Stromfluss auch, wenn er kein Betätigungssignal von einem Fußtaster oder von einem Spulentaster erhält. Damit wird sichergestellt, dass der magnetische Neurostimulators nicht ungewollt betrieben werden kann, sondern nur dann, wenn er bewusst "scharfgeschaltet" wird, indem ein Fußtaster oder ein Spulentaster betätigt wird.

Gemäß einer vorteilhaften Weiterbildung der Erfindung sind zwei Schließschalter vorgesehen. Dabei unterbricht der erste Schließschalter den Stromfluss der Stimulationsspule, wenn er ein Fehlersignal des ersten Oder-Schaltelements oder kein Betätigungssignal des Spulentasters erhält. Der zweite Schließschalter unterbricht den Stromfluss der Stimulationsspule, wenn er ein Fehlersignal des zweiten Oder-Schaltelementes oder kein Betätigungssignal des Fußtasters erhält. Zur Verknüpfung der Signale der Oder-Schaltelemente und der Betätigungssignale des Spulen- und Fußtasters können weiterhin Und-Schaltelemente sowie Inverter vorgesehen werden. Dabei verknüpft ein erstes Und-Schaltelement das durch den ersten Inverter invertierte Ausgangssignal des ersten Oder-Schaltelements mit dem Betätigungssignal des Spulentasters. Das zweite Und-Schaltelement verknüpft das durch den zweiten Inverter invertierte Ausgangssignal des zweiten Oder-Schaltelements mit dem Betätigungssignal des Fußtasters.

Gemäß einer weiteren Ausführungsform der Erfindung ist die Pulsquelle des magnetischen Neurostimulators als Pulskondensator ausgebildet, der mit der Stimulationsspule kurzzeitig einen Schwingkreis bildet, wenn der Schwingkreis durch den Pulsauslöserschalter geschlossen wird.

Das Aufladen des Pulskondensators kann durch eine zusätzlich vorsehbare Ladeschaltung erfolgen, die wenigstens eine Spannungsquelle aufweist. Zusätzlich dazu kann die Ladeschaltung optional einen zusätzlichen Ladekondensator und eine Halb- oder Vollbrücken-Ladeschaltung beinhalten.

Der Pulsauslöser-Schalter kann wenigstens einen Thyristor, zwei gegengleich parallelgeschaltete Thyristoren, einen Thyristor mit gegengleich geschalteter Freilaufdiode oder wenigstens einen IGBT bzw. Insulated Gate Bipolar Transistor aufweisen.

Die Erfindung betrifft auch ein Verfahren zur magnetischen Neurostimulation mit einer Ausgangswertüberwachung. Dabei wird zunächst ein vorstehend beschriebener magnetischer Neurostimulator mit einer vorstehend beschriebenen Ausgangswertüberwachungs-Schaltung bereitgestellt. Die Pulsquelle wird dabei zunächst aufgeladen. Dann werden durch einen Benutzer der Spannungsbeschränkungs-Wahlschalter und/oder der Pulsfrequenzbeschränkungs-Wahlschalter auf bestimmte Spannungs- und/oder Pulsfrequenz-Grenzwerte eingestellt. Anschließend werden der Pulsauslöserschalter geschlossen und durch die Stimulationsspule ein Magnetfeld zum Stimulieren des Kortex oder der Peripherie mit induziertem Wechselstrom erzeugt, und zwar mit einer durch die Pulsquelle vorgegebenen Intensität und mit einer durch den Pulsgenerator erzeugten Frequenz. Wenn eine an der Pulsquelle gemessene Spannung den Spannungsgrenzwert übersteigt und/oder wenn eine von dem Pulsgenerator vorgegebene Pulsfrequenz den Pulsfrequenz-Grenzwert übersteigt, wird ein optischer und/oder akustischer Alarm ausgegeben und alternativ oder zusätzlich dazu der Schwingkreis unterbrochen.

Dadurch wird zuverlässig erkannt und verhindert, dass zu hohe Spannungen oder Pulsfrequenzen auf den Patienten einwirken. Somit wird das Risiko für den Patienten, insbesondere das Risiko eines epileptischen Anfalls bei der magnetischen Neurostimulation deutlich vermindert.

Zusammenfassend ist festzustellen, dass aufgrund des unabhängigen Aufbaus die Ausgangswert-Überwachungsschaltung wirksam gegen Bedienfehler über die Frontplatte, gegen Ansteuerfehler durch externe Geräte und gegen geräteinterne Hard- und Softwarefehler in weiten Teilen des Aktionskanals ist. Dies wird dadurch erreicht, dass die Pulsbefehle schaltplantechnisch so spät wie möglich, also erst vor der Ansteuerung des/der Pulsschalter stattfindet. Eine fehleranfällige softwareseitige Unterbindung wird bei der vorliegenden Erfindung vermieden.

Die Erfindung ist nachfolgend anhand eines Ausführungsbeispiels mit Bezug auf die Figuren näher erläutert.
- Figur 1: zeigt ein Blockschaltbild eines Neurostimulator-Leistungsteils,
- Figur 2: zeigt ein Blockschaltbild einer Ausgangswert-Überwachungsschaltung eines Neurostimulators,
- Figur 3: zeigt eine Frontplatte eines Leistungsteil-Gehäuses des Neurostimulators.

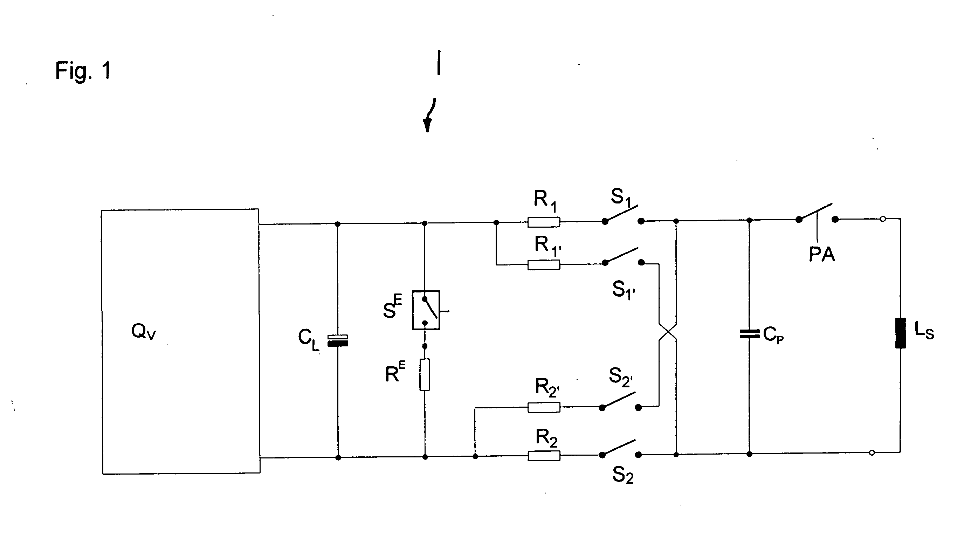

Figur 1 zeigt ein Blockschaltbild eines Neurostimulator-Leistungsteils 1. Das Neurostimulator-Leistungsteil 1 umfasst einen Schwingkreis aus einem Pulskondensator C_{P} und aus der Stimulationsspule bzw. externen Behandlungsspule L_{S}. der über den Pulsauslöserschalter PA geschlossen und unterbrochen werden kann. Der Pulsauslöserschaltung PA ist vorliegend als IGBT ausgeführt.

Die Stimulationsspule Ls wird beim Schließen des Pulsauslöseschalters PA von einem Strom durchflossen und erzeugt so ein zeitabhängiges Magnetfeld. Beim Einsatz des Neurostimulators wird die Stimulationsspule Ls über den Kopf eines Patienten geführt. Dabei depolarisiert das von dem zeitabhängigen Magnetfeld erzeugte elektrische Feld die Nerven im Kopf des Patienten. Der Pulskondensator Cp und die Stimulationsspule Lₛ bilden kurzzeitig einen Schwingkreis, an dem eine Spannung von 1000 bis 3000 Volt anliegt und ein Strom von 500 bis 8000 Ampere fließt.

Der in Figur 1 links von dem Schwingkreis angeordnete Schaltungsteil stellt eine Ladeschaltung für den Pulskondensator Cp dar. Diese Ladeschaltung verfügt über eine Spannungsquelle Qv und über zwei Brücken von Ladeschaltern S₁ und S₂ bzw. S_{1'} und S_{2'} mit den zugeordneten Vorwiderständen R₁ und R₂ bzw. R_{1'} und R_{2'}. Je nach Polung des Pulskondensators C_{P} werden für dessen Aufladung entweder das Schalterpaar S₁ und S₂ oder das Schalterpaar S_{1'} und S_{2'} geschlossen. Die Ladeschaltung in Figur 1 verfügt noch über einen optional vorsehbaren Ladekondensator C_{L} und über eine Entladeschleife mit einem Entladeschalter SE und mit einem Entladewiderstand R^{E}. Die Vorwiderstände R₁, R_{1'}, R₂ und R_{2'} sind optional und je nach verwendetem Typ der Ladeschalter S_{1,} S_{1',} S₂ und S_{2'} nicht notwendig. Die Ladeschalter S_{1,} S_{1',} S₂ und S_{2'} können als beliebige geeignete elektrische oder mechanische Schalter ausgeführt werden. Im Ausführungsbeispiel gemäß Figur 1 sind die Ladeschalter S₁, S_{1'}, S₂ und S_{2'} als IGBT's ausgeführt.

Die in Figur 1 dargestellte Vollbrücke der Ladeschaltung mit dem zweiten Schalterpaar S_{1'} und S_{2'} sowie mit den davor geschalteten Vorwiderständen R_{1'} und R_{2'} ist nur bei bestimmten Schaltungen für die Pulsauslösung nötig.

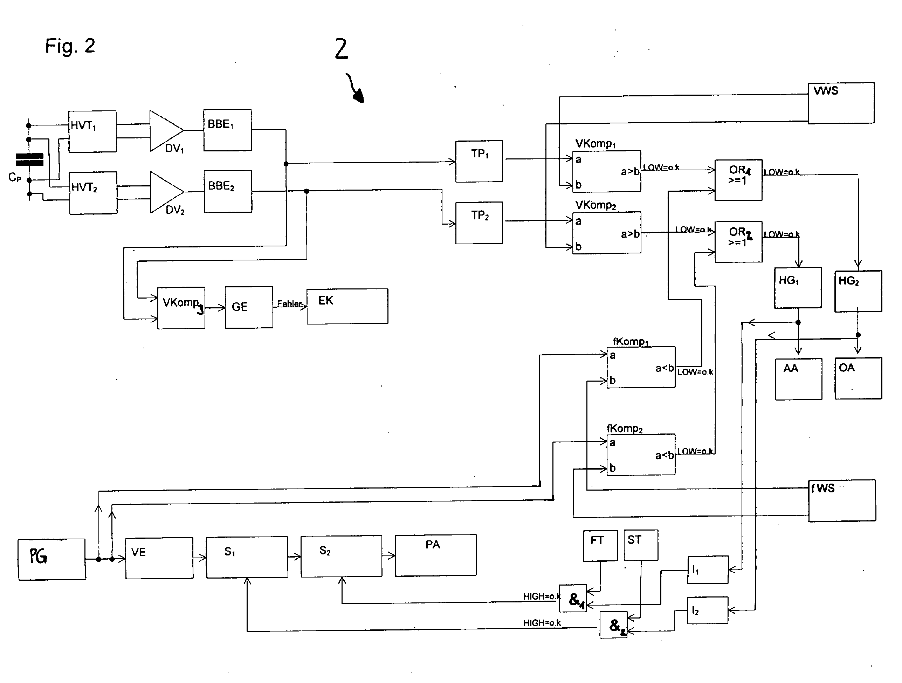

Figur 2 zeigt ein Blockschaltbild einer Ausgangswert-Überwachungsschaltung 1 eines Neurostimulators. Die in Figur 2 dargestellte Ausgangswert-Überwachungsschaltung 2 setzt an dem bereits in Figur 1 gezeigten Impulskondensator C_{P} an und zeigt mittig unten den Pulsauslöserschalter PA.

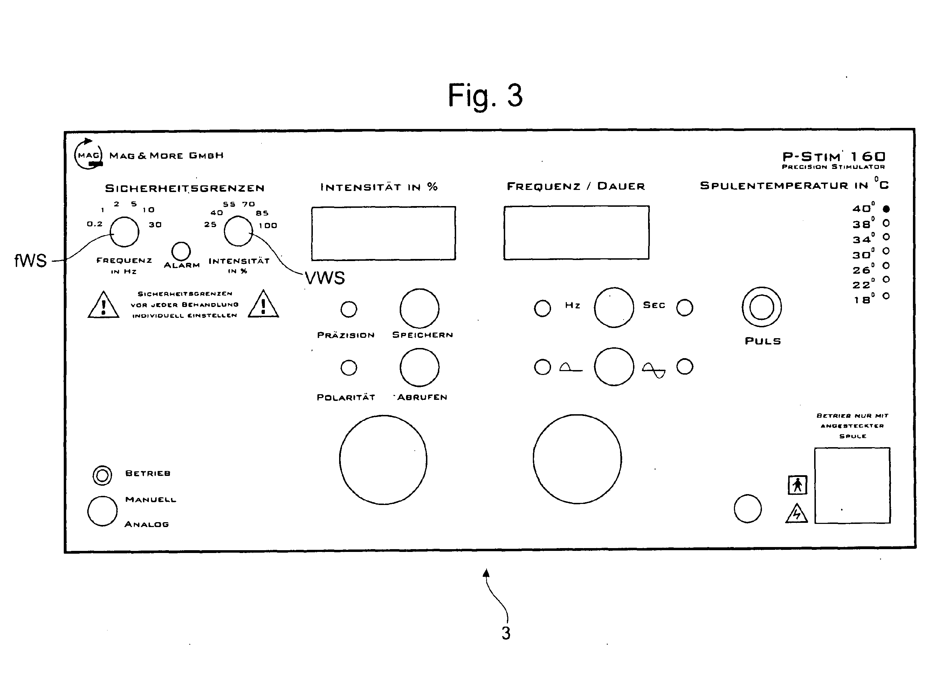

Des Weiteren verfügt die Ausgangswert-Überwachungsschaltung 2 über einen Spannungsbeschränkungs-Wahlschalter VWS und einen Pulsfrequenzbeschränkungs-Wahlschalter fWS, die auch in Figur 3 zu sehen sind. Der Spannungsbeschränkungs-Wahlschalter VWS und der Pulsfrequenzbeschränkungs-Wahlschalter fWS sind im vorliegenden Ausführungsbeispiel als Drehregler ausgeführt, die jeweils zwei Schaltebenen aufweisen.

Die übrigen Schaltungselemente der Ausgangswertüberwachungs-Schaltung 2 sind zusätzlich zu den in Figur 1 gezeigten Schaltungselementen vorzusehen.

Der obere Teil der Ausgangswert-Überwachungsschaltung 2 bildet eine Spannungsüberwachungs-Teilschaltung mit jeweils redundant ausgelegten Hochspannungsteilen HVT₁ und HVT₂, Differenzialverstärkern DV₁ und DV₂, Betragsbildungs-Schaltelementen BBE₁ und BBE₂, Tiefpassfiltern TP₁ und TP₂ und Spannungskomparatoren VKomp₁ und VKomp₂. Die hier zweikanalig ausgeführte Redundanz dient der Erhöhung der technischen Zuverlässigkeit, ist jedoch für die Funktion der Ausgangswert-Überwachungsschaltung 2 nicht notwendig. Die Spannungsteiler HVT₁ und HVT₂ erfassen jeweils die an dem Pulskondensator C_{P} anliegenden Ist-Spannungen A und B, die Differenzverstärker DV₁ und DV₂, die Betragsbildungsschaltelemente BBE₁ und BBE₂ sowie die Tiefpassfilter TP₁ und TP₂ bereiten die gemessenen Ist-Spannungen für die Spannungskomparatoren VKomp₁ und VKomp₂ auf.

In Figur 2 ist nach den Betragsbildungsschaltelementen BBE₁ und BBE₂ eine Komparator-Schaltung zur Plausibilitätsprüfung der Spannungswerte angeordnet. Diese verfügt über einen dritten Spannungskomparator VKomp₃ und über ein Glättungsschaltelement GE, dass im Falle eines festgestellten Fehlers den Schwingkreis unterbricht und auf einen Entladekanal EK schaltet. Diese Komparatorschaltung dient der Kontrolle der Spannungserfassung und damit der Erhöhung der technischen Zuverlässigkeit.

Die derart gemessenen und aufbereiteten Spannungswerte werden durch die Spannungskomparatoren VKomp₁ und VKomp₂ mit Spannungs-Grenzwerten b verglichen, die an dem Spannungsbeschränkungs-Wahlschalter VWS eingestellt worden sind. Wenn die gemessenen und aufbereiteten Spannungswerte kleiner sind als der Spannungs-Grenzwert, so erfolgt die Ausgabe eines "O.K."-Signals, das im vorliegenden Ausführungsbeispiel gleich "low" ist. Übersteigen die gemessenen und aufbereiteten Spannungswerte den Spannungs-Grenzwert, so schalten die Spannungskomparatoren VKomp₁ und VKomp₂ ihren Ausgang auf den Fehlerwert "high".

Die Pulsfrequenzüberwachungs-Teilschaltung verfügt über einen internen Pulsgenerator PG und über zwei ebenfalls redundant ausgelegte Frequenzkomparatoren fKomp₁ und fKomp₂, welche die von dem Pulsgenerator PG angelegte Pulsfrequenz bzw. Pulsfolge auf Überschreiten des durch den Pulsfrequenzbeschränkungs-Wahlschalter fWS festgelegten Pulsfrequenz-Grenzwert überprüft. Falls die erzeugte Frequenz darunter liegt und die Pulsfolge demnach die durch den Pulsfrequenz-Grenzwert vorgegebene Maximalfrequenz von b einhält, so wird von den Frequenzkomparatoren fKomp₁ und fKomp₂ ein "O.K."-Wert ausgegeben, der im vorliegenden Ausführungsbeispiel ein "low"-Wert ist. Übersteigt die erzeugte Frequenz den Pulsfrequenz-Grenzwert, so geben die Frequenzkomparatoren fKomp₁ und fKomp₂ den Fehlerwert "high" aus.

Die Logikpegel sind in Figur 2 beispielhaft gewählt, sie können grundsätzlich auch invertiert werden, sodass anstelle des "low"-Werts ein "high"-Wert als "O.K."-Wert gewählt wird.

Die Ausgangswerte der Spannungskomparatoren VKomp₁ und VKomp₂ sowie der Frequenzkomparatoren fKomp₁ und fKomp₂ bilden die Eingänge der ebenfalls redundant vorgesehenen Oder-Gatter OR₁ und OR₂. Wenn an den Eingängen der Oder-Gatter OR₁ und OR₂ "low"-Werte anliegen, so werden von den Oder-Gattern OR₁ und OR₂ jeweils "low"-Werte ausgegeben. Ist jedoch wenigstens eines der an den Eingängen anliegenden Komparator-Ausgangssignale ein fehlerhaftes "high"-Signal, so wird der Ausgang der Oder-Gatter OR₁ und OR₂ ebenfalls auf "high" geschaltet. In diesem Fall halten die nach den Oder-Gattern OR₁ und OR₂ angeordneten Halteglieder HG₁ und HG₂ das fehlerhafte Signal während einer vorbestimmten Haltezeit, die im vorliegenden Ausführungsbeispiel 5 Sekunden beträgt. Die Ausgangssignale der Halteglieder HG₁ und HG₂ aktivieren jeweils einen akustischen Alarmgeber AA, beispielsweise einen Summer oder einen optischen Alarmgeber OA, beispielsweise eine LED. Diese Alarmgeber AA und OA geben solange einen Alarm aus, solange an ihrem Eingang ein Wert "high" anliegt.

Die Ausgangswerte der Halteglieder HG₁ und HG₂ werden ferner an Inverter I₁ und I₂ weitergeleitet, die sie invertieren und an zwei Und-Gatter &₁ und &₂ weiterleiten. Dort werden sie mit Signalen von einem Fußtaster FT und einem Spulentaster ST verknüpft. Die zwei Und-Gatter &₁ und &₂ steuern mit ihren Ausgangswerten jeweils die Schließschalter S₁ und S₂. Die Schließschalter S₁ und S₂ empfangen das vom Pulsgenerator PG erzeugte Pulssignal, das von der Verzögerungseinheit VE um einen grundsätzlich beliebigen Wert, der im vorliegenden Ausführungsbeispiel 10 µs beträgt, verzögert worden ist, damit der Ausgangswertüberwachungs-Schaltung 2 ausreichend Zeit zur Verfügung steht, vom Pulsgenerator PG erzeugte Pulssignale, welche den zulässigen Pulsfrequenz-Grenzwert übersteigen und daher unzulässig sind, zu blockieren, bevor diese Pulssignale zur Auslösung kommen. Die Schließschalter Sᵢ und S₂ unterbrechen den Schwingkreis durch den danach angeordneten Pulsauslöserschalter PA, wenn sie von den Und-Gattern &₁ und &₂ ein "high"-Signal empfangen, das ein Überschreiten des Spannungs-Grenzwerts durch die am Pulskondensator CP gemessene Spannung, ein Überschreiten des Pulsfrequenz-Grenzwerts durch den vom Pulsgenerator PG erzeugten Stimulationspuls oder eine Unterbrechung der Betätigung des Fußtasters FT oder des Spulentasters ST anzeigt.

Durch die in Figur 2 gezeigte Ausgangswert-Überwachungsschaltung 2 wird sichergestellt, dass an der Stimulationsspule L_{S} keine zu hohen Spannungen oder Frequenzen anliegen. Die Spannungs- und Pulsfrequenz-Grenzwerte können mit den Wahlschaltern VWS und fWS vor der Behandlung des Patienten individuell eingestellt werden. Durch die Ausgangswertüberwachungs-Schaltung 2 ist sichergestellt, dass diese individuell eingestellten Grenzwerte bei der Behandlung nicht überschritten werden.

Die in Figur 2 dargestellte Ausgangswertüberwachungs-Schaltung 2 gibt erstens akustische oder optische Warnsignale aus und unterbricht zweitens die Abgabe von Stimulationspulsen, wenn fehlerhafte Spannungs- oder Frequenzwerte festgestellt werden. Die Ausgangswertüberwachungs-Schaltung 2 kann jedoch auch derart ausgestaltet sein, dass sie nur Signale abgibt oder dass sie nur die Pulsgabe unterbricht. In diesem Fall sind die in Figur 2 dargestellten, nicht benötigten Schaltungselemente wegzulassen. Ebenso kann die Ausgangswertüberwachungs-Schaltung 2 auch ohne Einschleifen von Signalen des Fußtasters FT und des Spulentasters ST ausgeführt werden.

Figur 3 zeigt eine Frontplatte 3 eines Leistungsteil-Gehäuses des Neurostimulators. In der Frontplattendarstellung gemäß Figur 3 sind neben Anzeigefeldern sowie Einstellreglern für die (Spannungs-) Intensität in Prozent und für die (Schwing-) Frequenz/Dauer und neben einem Anzeigefeld für die Spulentemperatur der Spannungsbeschränkungs-Wahlschalter VWS und der Pulsfrequenzbeschränkungs-Wahlschalter fWS besonders gut zu sehen.

### Bezugszeichenliste

- 1: Neurostimulator-Leistungsteil
- Qᵥ: Spannungsquelle
- C_{L}: Ladekondensator
- SE: Entladeschalter
- RE: Entladewiderstand
- R₁, R_{1'}, R₂, R_{2'}: Vorwiderstände
- S₁, S_{1'}, S₂, S_{2'}: Ladeschalter
- C_{P}: Pulskondensator
- PA: Pulsauslöserschalter
- Ls: Stimulationsspule
- 2: Ausgangswert-Überwachungsschaltung
- HVT₁, HVT₂: Hochspannungsteiler
- DV₁, DV₂: Differentialverstärker
- BBE₁, BBE₂: Betragsbildungs-Schaltelemente
- VKomp₁, VKomp₂, VKomp₃: Spannungskomparatoren
- TP₁, TP₂: Tiefpassfilter
- GE: Glättungs-Schaltelement
- EK: Entladekanal
- VWS: Spannungsbeschränkungs-Wahlschalter
- OR₁, OR₂: Oder-Gatter
- HG₁, HG₂: Halteglieder
- AA: akustischer Alarmgeber
- OA: optischer Alarmgeber
- fWS: Pulsfrequenzbeschränkungs-Wahischalter
- fKomp₁, fKomp₂: Frequenzkomparatoren
- FT: Fusstaster
- ST: Spulentaster
- I₁, I₂: Inverter
- &₁, &₂: Und-Gatter
- PG: Pulsgenerator
- VE: Verzögerungseinheit
- S₁, S₂: Schließschalter
- 3: Frontplatte

## Patentansprüche

1. Magnetischer Neurostimulator mit einer Ausgangswert-Überwachungsschaltung, wobei der magnetische Neurostimulator die folgenden Merkmale aufweist:
- einen Schwingkreis mit einer Stimulationsspule (Ls) zum Erzeugen eines Magnetfelds und mit einer Pulsquelle (C_{P}), die eine Spannung an die Stimulationsspule (Ls) abgibt;
- einen Pulsgenerator (PG) zum Generieren eines Pulsauslösersignals;
- einen Pulsauslöserschalter (PA), der in Abhängigkeit des Pulsauslösersignals den Schwingkreis schliesst oder unterbricht;
- einen Spannungsbeschränkungs-Wahlschalter (VWS) zum Einstellen eines Spannungs-Grenzwerts; und/oder
- einen Pulsfrequenzbeschränkungs-Wahlschalter (fWS) zum Einstellen eines Pulsfrequenz-Grenzwerts;
- eine Ausgangswert-Überwachungsschaltung (2), die einen optischen und/oder akustischen Alarm ausgibt und/oder die den Schwingkreis unterbricht, wenn eine an der Pulsquelle (C_{P}) gemessene Spannung den Spannungs-Grenzwert übersteigt und/oder wenn eine von dem Pulsgenerator (PG) vorgegebene Pulsfrequenz den Pulsfrequenz-Grenzwert übersteigt.

2. Magnetischer Neurostimulator nach Anspruch 1,
**dadurch gekennzeichnet, dass**
die Ausgangswert-Überwachungsschaltung (2) jeweils eine Spannungsüberwachungs-Teilschaltung zum Erfassen und zum Aufbereiten der Spannung und eine Pulsfrequenzüberwachungs-Teilschaltung aufweist.

3. Magnetischer Neurostimulator nach Anspruch 2,
**dadurch gekennzeichnet, dass**
die Spannungsüberwachungs-Teilschaltung wenigstens einen Spannungskomparator (VKomp₁, VKomp₂) aufweist, dessen erster Eingang einen Spannungswert von der Pulsquelle (C_{P}) erhält und dessen zweiter Eingang mit dem Spannungsbeschränkungs-Wahlschalter (VWS) in Verbindung steht.

4. Magnetischer Neurostimulator nach Anspruch 3,
**dadurch gekennzeichnet, dass**
die Spannungsüberwachungs-Teilschaltung zum Aufbereiten der gemessenen Spannungswerte wenigstens einen an der Pulsquelle (Cp) ansetzenden Hochspannungsteiler (HVT₁, HVT₂), wenigstens einen danach angeordneten Differenzverstärker (DV₁, DV₂) und wenigstens ein danach angeordnetes Betragsbildungs-Schaltelement (BSE₁, BBE₂), das mit dem ersten Eingang des Spannungskomparators (VKomp₁, VKomp₂) verbunden ist.

5. Magnetischer Neurostimulator nach Anspruch 4,
**dadurch gekennzeichnet, dass**
weiterhin wenigstens ein Tiefpassfilter (TP₁, TP₂) vorgesehen ist, der sich an das Betragsbildungs-Schaltelement (BBE₁, BBE₂) anschließt und der mit dem ersten Eingang des Spannungskomparators (VKomp₁, VKomp₂) verbunden ist.

6. Magnetischer Neurostimulator nach Anspruch 4 oder 5,
**dadurch gekennzeichnet, dass**
die Spannungsüberwachungs-Teilschaltung über zwei redundante, parallel angeordnete Schaltungsarme verfügt, die jeweils einen Hochspannungsteiler (HVT₁, HVT₂), einen Differenzverstärker (DV₁, DV₂), ein Betragsbildungs-Schaltelement (BBE₁, BBE₂), einen Tiefpassfilter (TP₁, TP₂) und einen Spannungskomparator (VKomp₁, VKomp₂) aufweisen.

7. Magnetischer Neurostimulator nach Anspruch 6,
**dadurch gekennzeichnet, dass**
nach den Betragsbildungs-Schaltelementen (BBE₁, BBE₂) eine Komparatorschaltung (VKomp₃) zur Plausibilitätsprüfung der Spannungswerte angeordnet ist, welche die von den parallel angeordneten Hochspannungsteilern (HVT₁, HVT₂), Differenzverstärkern (DV₁, DV₂) und Betragsbildungs-Schaltelementen (BBE₁, BBE₂) gemessenen und aufbereiteten Spannungswerte vergleicht und den Schwingkreis unterbricht, falls ein Fehler festgestellt wird.

8. Magnetischer Neurostimulator nach Anspruch 7,
**dadurch gekennzeichnet, dass**
die Komparatorschaltung (VKomp₃) die gemessenen und aufbereiteten Spannungswerte glättet.

9. Magnetischer Neurostimulator nach einem der Ansprüche 2 bis 8,
**dadurch gekennzeichnet, dass**
die Pulsfrequenzüberwachungs-Teilschaltung wenigstens einen Pulsfrequenzkomparator (fKomp₁. fKomp₂) aufweist, dessen erster Eingang von dem Pulsgenerator (PG) einen Stimulationspuls erhält und dessen zweiter Eingang mit dem Pulsfrequenzbeschränkungs-Wahlschalter (fWS) in Verbindung steht.

10. Magnetischer Neurostimulator nach Anspruch 9,
**dadurch gekennzeichnet, dass**
die Pulsfrequenzüberwachungs-Teilschaltung über zwei redundante, parallel angeordnete Pulsfrequenzkomparatoren (fKomp₁, fKomp₂) verfügt.

11. Magnetischer Neurostimulator nach Anspruch 9 oder 10,
**dadurch gekennzeichnet, dass**
die Spannungsüberwachungs-Teilschaltung und die Pulsfrequenzüberwachungs-Teilschaltung über wenigstens ein Oder-Schaltelement (OR₁, OR₂) mit wenigstens einem akustischen oder optischen Alarmgeber (AA; OA) verbunden sind, der durch ein Fehlersignal des Oder-Schaltelements (OR₁, OR₂) aktivierbar ist.

12. Magnetischer Neurostimulator nach Anspruch 11,
**dadurch gekennzeichnet, dass**
bei einer redundanten Auslegung der Spannungsüberwachungs-Teilschaltung und der Pulsfrequenzüberwachungs-Teilschaltung zwei Oder-Schaltelemente (OR₁, OR₂) vorhanden sind.

13. Magnetischer Neurostimulator nach Anspruch 11 oder 12,
**dadurch gekennzeichnet, dass**
nach dem Oder-Schaltelement bzw. nach den Oder-Schaltelementen (OR₁, OR₂) jeweils ein Halteglied (HG₁, HG₂) vorgesehen ist, welches das Fehlersignal des betreffenden Oder-Schaltelements (OR₁, OR₂) für einen vorbestimmten Zeitraum hält.

14. Magnetischer Neurostimulator nach einem der Ansprüche 11 bis 13,
**dadurch gekennzeichnet, dass**
nach dem Pulsgenerator (PG) ein Verzögerungselement (VE) vorgesehen ist.

15. Magnetischer Neurostimulator nach Anspruch 14,
**dadurch gekennzeichnet, dass**
nach dem Verzögerungselement (VE) wenigstens ein Schließschalter (S₁, S₂) vorgesehen ist, der den Schwingkreis unterbricht, wenn er ein Fehlersignal des bzw. eines Oder-Schaltelements (OR₁, OR₂) erhält.

16. Magnetischer Neurostimulator nach Anspruch 15,
**dadurch gekennzeichnet, dass**
der bzw. ein Schließschalter (S₁, S₂) den Stromfluss der Stimulationsspule (Ls) auch dann unterbricht, wenn er ein Betätigungssignal von einem Fusstaster (FT) oder von einem Spulentaster (ST) erhält bzw. nicht erhält.

17. Magnetischer Neurostimulator nach Anspruch 16,
**dadurch gekennzeichnet, dass**
zwei Schließschalter (S₁, S₂) vorgesehen sind, wobei der erste Schließschalter (Si) den Stromfluss der Stimulationsspule (L_{S}) dann unterbricht, wenn er ein Fehlersignal des ersten Oder-Schaltelements (OR₁) oder kein Betätigungssignal des Spulentasters (ST) erhält und wobei der zweite Schließschalter (S₂) den Stromfluss der Stimulationsspule (L_{S}) dann unterbricht, wenn er ein Fehlersignal des zweiten Oder-Schaltelements (OR₂) oder kein Betätigungssignal des Fusstasters (FT) erhält.

18. Magnetischer Neurostimulator nach Anspruch 17,
**dadurch gekennzeichnet, dass**
ein erstes Und-Schaltelement (&₁) vorgesehen ist, welches das durch einen ersten Inverter (I₁) invertierte Signal des ersten Oder-Schaltelements (OR₁) mit dem Betätigungssignal des Spulentasters (ST) verknüpft, und dass ein zweites Und-Schaltelement (&₂) vorgesehen ist, welches das durch einen zweiten Inverter (I₂) invertierte Signal des zweiten Oder-Schaltelements (OR₂) mit dem Betätigungssignal des Fusstasters (FT) verknüpft.

19. Magnetischer Neurostimulator nach einem der vorhergehenden Ansprüche,
**dadurch gekennzeichnet, dass**
die Pulsquelle (C_{P}) als Pulskondensator (C_{P}) ausgebildet ist.

20. Magnetischer Neurostimulator nach Anspruch 19,
**dadurch gekennzeichnet, dass**
der Pulskondensator (C_{P}) eine Ladeschaltung mit wenigstens einer Spannungsquelle (Q_{V}) aufweist.

21. Magnetischer Neurostimulator nach Anspruch 20,
**dadurch gekennzeichnet, dass**
die Ladeschaltung einen zusätzlichen Ladekondensator (C_{L}) und eine Halb- oder Vollbrücken-Ladeschaltung aufweist.

22. Magnetischer Neurostimulator nach einem der vorhergehenden Ansprüche,
**dadurch gekennzeichnet, dass**
der Pulsauslöserschalter (PA) wenigstens einen Thyristor aufweist.

23. Magnetischer Neurostimulator nach Anspruch 22,
**dadurch gekennzeichnet, dass**
der Pulsauslöserschalter (PA) zwei gegengleich parallelgeschaltete Thyristoren aufweist.

24. Magnetischer Neurostimulator nach Anspruch 23,
**dadurch gekennzeichnet, dass**
der Pulsauslöserschalter (PA) einen Thyristor mit gegengleich geschalteter Freilaufdiode aufweist.

25. Magnetischer Neurostimulator nach einem der Ansprüche 1 bis 21,
**dadurch gekennzeichnet, dass**
der Pulsauslöserschalter (PA) wenigstens einen IGBT aufweist.

26. Verfahren zur magnetischen Neurostimulation mit einer Ausgangswert-Überwachung mit den folgenden Schritten:
- Bereitstellen eines magnetischer Neurostimulators mit einer Ausgangswert-Überwachungsschaltung (2) nach einem der Ansprüche 1 bis 23;
- Einstellen des Spannungsbeschränkungs-Wahlschalters (VWS) auf einen bestimmten Spannungs-Grenzwert und/oder des Pulsfrequenzbeschränkungs-Wahlschalters (fWS) auf einen bestimmten Frequenz-Grenzwert;
- Schließen des Pulsauslöserschalters und Erzeugen eines Magnetfelds durch die Stimulationsspule (Ls) mit einer durch die Pulsquelle (C_{P}) vorgegebenen Intensität und mit einer durch den Pulsgenerator (PG) erzeugten Frequenz;
- Ausgeben eines optischen und/oder akustischen Alarms und/oder Unterbrechen des Schwingkreises, wenn eine an der Pulsquelle (Cp) gemessene Spannung den Spannungs-Grenzwert übersteigt und/oder wenn eine von dem Pulsgenerator (PG) vorgegebene Pulsfrequenz den Pulsfrequenz-Grenzwert übersteigt.
